# EUROPEAN PATENT SPECIFICATION

(11) **EP 3 245 297 B1**
(45) Date of publication and mention of the grant of the patent: **01.01.2020**
(21) Application number: 15744709.5
(22) Date of filing: 11.06.2015
(51) Int. Cl.: C12Q 1/6888

(54) **METHOD AND KIT FOR IDENTIFICATION OF NEMATODES, FOREST PLANT PESTS, BY REAL-TIME PCR**
VERFAHREN UND KIT ZUM NACHWEIS VON NEMOTODEN, FORSTLICHE PFLANZENSCHÄDLINGE, MITTELS ECHTZEIT-PCR
MÉTHODE ET KIT POUR L'IDENTIFICATION DE NÉMATODES, NUISIBLES AUX PLANTES FORESTIÈRES, PAR PCR EN TEMPS RÉEL

(30) Priority: 16.01.2015 PL 41098215
(43) Date of publication of application: 22.11.2017
(73) Proprietor: Muzeum i Instytut Zoologii Polskiej Akademii Nauk, 00-679 Warszawa (PL)
(72) Inventor: BOGDANOWICZ, Wieslaw, 02-547 Warszawa (PL); DMOWSKA, Ewa, 01-493 Warszawa (PL); FLIS, Lukasz, 05-334 Latowicz (PL); GRALAK, Aleksandra, 21-400 Luków (PL); ILIEWA-MAKULEC, Krassimira, 03-379 Warszawa (PL); KOWALEWSKA, Katarzyna, 04-174 Warszawa (PL); LOS, Marta, 01-946 Warszawa (PL); POMORSKI, Jan Jakub, 02-134 Warszawa (PL); RYBARCZYK-MYDLOWSKA, Katarzyna, 00-160 Warszawa (PL); RYCHLICKA, Edyta, 64-920 Pila (PL); TOMALAK, Marek, 61-044 Poznan (PL); TURLEJ, Robert, 01-310 Warszawa (PL); WISNIEWSKA, Katarzyna, 00-538 Warszawa (PL); WISNIEWSKA, Olga, 02-681 Warszawa (PL); MALEWSKI, Tadeusz, 03-528 Warszawa (PL); SKWIERCZ, Andrzej, 81-363 Gdynia (PL); TEREBA, Anna, 05-806 Kormorów (PL); WINISZEWSKA-SLIPINSKA, Grazyna, 03-436 Warszawa (PL)
(74) Representative: Kondrat, Mariusz
(86) International application number: PCT/PL2015/000092
(87) International publication number: WO 2016/114675

(56) References cited:
- WO-A2-2004/090164
- KR-A- 20100 019 645
- KANG JAE SOON ET AL: "Rapid and accurate prediction of the frequencies of Bursaphelenchus xylophilus and B. mucronatus in mixed nematode samples using real-time species-specific PCR", NEMATOLOGY, BRILL, LEIDEN, NL, vol. 11, no. 2, 1 January 2009 (2009-01-01), pages 289-299, XP009186050, ISSN: 1388-5545
- FILIPE PEREIRA ET AL: "New Insights into the Phylogeny and Worldwide Dispersion of Two Closely Related Nematode Species, Bursaphelenchus xylophilus and Bursaphelenchus mucronatus", PLOS ONE, vol. 8, no. 2, 8 February 2013 (2013-02-08), page e56288, XP055213362, DOI: 10.1371/journal.pone.0056288

## Description

The object of the invention is a method and a kit for identification of nematodes, forest plant pests, by Real-Time PCR. In particular, the invention relates to a method and a kit for identification of nematodes selected from the group consisting of the species *Bursaphelenchus mucronatus* from the family Aphelenchoididae, *Mesocriconema xenoplax* from the family Criconematidae, and *Rotylenchus uniformis* from the family Hoplolaiminae.

*Bursaphelenchus xylophilus* is a conifer parasite and a dangerous conifer pest causing the so- called 'pine wilt' disease. It multiplies living in resin ducts. This leads to their obstruction and as a result to the plant's death. *B. xylophilus* is vectored by various insects developing in wood infested by it, especially those of the family of longicorns (Cerambycidae). It is considered a quarantine pest. *Bursaphelenchus mucronatus,* as *B. xylophilus,* belongs to the 'xylophilus' group within the *Bursaphelenchus* genus. Identification of nematode species is based on analysis of morphological and morphometric features, including diagnosis of inter alia: female coloration at various developmental stages, cyst shape, stylet length and knob shape, invasive larvae length, perineal pattern. However, this analysis is very laborious and time-consuming. It requires extensive knowledge and experience in working with biological material. Moreover, there is a possibility of size overlapping for different species. The method based on analysis of morphological and morphometric features cannot be used to identify juvenile specimens, not yet having the morphological features developed.

Currently in nematological diagnostics, use of techniques based on nucleic acid analysis is increasing, including those employing polymerase chain reaction (PCR). The principle of PCR is amplification of DNA fragment(s), specific to the given organism, up to a level allowing a rapid and simple detection thereof using electrophoresis. This is achieved by using short singlestranded oligonucleotides (12-40 nucleotides), the so called primers, specific to the amplified DNA fragment and an enzyme - a thermostable polymerase, enabling amplification of the desired fragment in a cyclic three-stage reaction, composed of denaturation, primer annealing and DNA synthesis. Typically after approximately 30 reaction cycles over a million copies of the amplified DNA fragment is obtained, allowing its identification with gel electrophoresis.

An improvement for the polymerase chain reaction is Real Time PCR, being a PCR reaction with a measurement of the amplified fragment quantity in each reaction cycle. In order to measure the amplified fragment quantity fluorochromes (fluorescent dyes) are used, e.g.: SYBR Green I, SYTO9, Eva Green, SYBR Gold, fluorescence of which is proportional to the quantity of the amplified fragment. The resulting products identification is done through measuring fluorescence intensity and melting curve analysis for the reaction products without electrophoresis. The sensitivity of Real Time PCR is much greater than that of traditional PCR and the absence of electrophoresis reduces analysis time. A drawback of Real Time PCR with fluorescent dyes (as for the traditional PCR) is limited reaction specificity. In most cases, the PCR reaction occurs not only for 100% primer sequence identity with the template sequence but also when 1-2 nucleotides are not identical. This characteristic of traditional PCR and Real Time PCR with fluorescent dyes requires precise setup of thermal parameters for the reaction. Furthermore, fluorescent dyes bind to every double-stranded DNA fragment, also the ones resulting from primer amplification (the primer-primer, primer-dimer products), which requires careful selection of suitable primer concentration.

An alternative for fluorescent dyes are fluorescently labeled DNA probes. They are short DNA fragments, complementary to the sequence of interest, emitting fluorescence at a particular wavelength upon binding DNA during the PCR reaction. Currently several types of probes are known, e.g.: TaqMan, FRET, molecular beacons or scorpions. The specificity of Real Time PCR with labeled probes is significantly higher than with fluorescent dyes. In contrast to primers, a mismatch of a single nucleotide in the probe sequence typically leads to no reaction or a significant decrease in efficiency, being easy to detect while monitoring the course of the reaction or by analyzing reaction results.

The hitherto most frequently used method of molecular identification of nematodes was PCR. McCuiston J.L, Hudson L.C. Subbotin S.A., Davis E.L., Warfield C.Y Conventional and PCR Detection of Aphelenchoides fragariae in Diverse Ornamental Host Plant Species. J Nematol. Dec 2007; 39(4): 343-355 designed primers for identification of *Aphelenchoides fragariae.*

A Restriction Fragments Length Polymorphism (PCR-RFLP) method was used, utilizing restriction enzymes cleaving a DNA strand at their specific locations. The differences in length of the cleaved DNA fragments indicate variability. Burgermeister W., Metge K., Braasch H., Buchbach E. 2005. ITS-RFLP patterns for differentiation of 26 Bursaphelenchus species (Nematoda: Parasitaphelenchidae) and observations on their distribution. Russian Journal of Nematology, 13: 29-42 have developed a PCR-RFLP method for identification of 44 *Bursaphelenchus* species, including identification of *Bursaphelenchus mucronatus.* Identification of *B. mucronatus* with a PCR-RFLP method was also described in the publication of Zheng J, Subbotin SA, He S, Gu J, Moens M. 2003. Molecular characterisation of some Asian isolates of Bursaphelenchus xylophilus and B. mucronatus using PCR-RFLPs and sequences of ribosomal DNA. Russian Journal of Nematology 11 (1): 17-22.

For the identification of *B. xylophylus* a Real Time PCR method was successfully used (Cao AX, Liu XZ, Zhu SF, Lu BS. Detection of the Pinewood Nematode, Bursaphelenchus xylophilus, Using a Real-Time Polymerase Chain Reaction Assay. Phytopathology. 2005 May;95(5):566- 571; Ye W, Giblin-Davis RM. Molecular characterization and development of real-time PCR assay for pine-wood nematode Bursaphelenchus xylophilus (Nematoda: Parasitaphelenchidae). PLoS One. 2013 Nov 11;8(11):e78804. doi:10.1371/journal.pone.0078804).

Identification of *Mesocriconema xenoplax* by DNA sequencing was described in the publication of Cordero MA, Robbins RT, Szalanski AL. 2012. Taxonomic and Molecular Identification of Mesocriconema and Criconemoides Species (Nematoda: Criconematidae). Journal of Nematology 44(4):399-426. Likewise, identification of *Rotylenchus uniformis* by DNA sequencing was described in works of Bae CH, Szalanski AL, Robbins RT. 2009 Phylogenetic Analysis of the Hoplolaiminae Inferred from Combined D2 and D3 Expansion Segments of 28S rDNA. Journal of Nematology 41(1):28-34. and Cantalapiedra-Navarretea C, Navas-Cortesa JA, Liebanasb G, Vovlasc N, Subbotin SA, Palomares-Riusa JE, Castillo P. 2013. Comparative molecular and morphological characterisations in the nematode genus Rotylenchus: Rotylenchus paravitis n. sp., an example of cryptic speciation. Zoologischer Anzeiger 252: 246-268.

In the KR 2010 0019645 a method for analyzing *Bursaphelenchus xylophilus* and *Bursaphelenchus mucronatus* through Real-Time PCR is disclosed. The method comprises: a step of analyzing sequence of 5s rRNA of *Bursaphelenchus xylophilus* and *Bursaphelenchus mucronatus* using a database; a step of culturing the *Bursaphelenchus xylophilus* and *Bursaphelenchus mucronatus*; a step of extracting DNA from the *Bursaphelenchus xylophilus* and *Bursaphelenchus mucronatus*; a step of analyzing a sequence; a step of designing a primer/probe; and a step of confirming specific and sensitivity through Real-Time PCR. Document KR 2010 0019645 doesn't discloses i.a. that the nematodes are identified in a soil sample.

WO 2004/090164 discloses a use of at least one of the single nucleotide polymorphisms (SNPs) of a ribosomal nucleic acid indicated in the description as a marker for detecting lower taxonomic groups of nematodes, for then determining a life strategy of a nematode, for then determining the biodiversity of a soil sample, and/or for then determining the soil health. The document describes a method for detecting a lower taxonomic group of nematodes in a sample, comprising the steps of : a) providing a ribosomal nucleic acid sample of the nematodes present in said sample; b) detecting a ribosomal nucleic acid with at least one single nucleotide polymorphism (SNP) according to Table 7 of the description in said ribosomal nucleic acid sample for demonstrating the presence of the respective lower taxonomic group of nematodes in said sample. WO application discloses also a kit for carrying out the method, comprising a composition comprising a specific binding partner for the polynucleotide isolated from nematode ribosomal nucleic acid, which polynucleotide has a length of between 6 and 50 nucleotides, preferably between 8 and 40 nucleotides, more preferably between 10 and 35 nucleotides, and which polynucleotide contains at least one SNP according to Table 7, and one or more of the parts chosen from the group consisting of DNA extraction means, DNA amplification means, DNA hybridization means, DNA ligation means and DNA detection means. No information about a Real Time of PCR reaction used in the method is indicated in the WO application.

There is still a need in the state of the art for tools enabling detection of selected nematode species not identified with genetic methods, as well as for an improved method of identification of those nematode species, which are detected with known genetic techniques not providing high accuracy and not excluding errors in the analysis.

The aim of the invention is to provide a method enabling identification of nematode species with high sensitivity and specificity. The aim of the invention is also to provide a method for identification of nematodes, hitherto not being detected with methods analyzing the studied species genetic material. The aim of the invention is also to provide new sequences of nucleotide primers and probes or an unobvious selection of known sequences and probes applicable in a method of nematode detection using a Real-Time PCR method, regardless of the analyzed sample type, their origin and purpose, as well as developmental stage.

The abovementioned aims are realized by the present invention.

The object of the invention is a method of identification of nematodes in a soil sample, selected from the group consisting of the species *Bursaphelenchus mucronatus, Mesocriconema xenoplax* and *Rotylenchus uniformis,* the method comprising the following stages:
a) providing a soil sample containing nematodes for identification;
b) washing the nematodes off the soil;
c) DNA isolation;
d) conducting a Real Time PCR reaction, using a pair of 3' and 5' primers and a probe;
e) comparing the amplification curve of the analyzed sample with amplification curves of a blank sample and negative control,
wherein for identification of one of the abovementioned species in the Real-Time PCR reaction, the used 3' and 5' primer and the probe suitable for the particular species are selected from the following list:

| Species | 5' primer | | 3' primer | | Probe | |
|---|---|---|---|---|---|---|
| | Name | Sequence | Name | Sequence | Name | Sequence |
| *Bursaphelenchus mucronatus* | Bmucfv | gatgcgtgtttagag gac | Bmucrv | ggaagaacgtagagca ca | Bmuc | aacaccgacccacccg ataa |
| *Mesocriconema xenoplax* | Mefv | cttgctcgtactggtc tg | Mxerv | cagcttctacaccgaga g | Mxe | cctgctcgtgctgttgtcaa |
| *Rotylenchus uniformis* | Runfv | acggaccaaggag tttag | Runrv | cccaggatcacacatcag | Run | cagggggagaccttcactt tcatt |

The object of the invention is also a kit for identification of nematodes selected from the group consisting of the species *Bursaphelenchus mucronatus*, *Mesocriconema xenoplax* and *Rotylenchus uniformis,* with a method described above, comprising the 3' and 5' primers and the probe suitable for the particular species, selected from the following list:

| Species | 5' primer | | 3' primer | | Probe | |
|---|---|---|---|---|---|---|
| | Name | Sequence | Name | Sequence | Name | Sequence |
| *Bursaphelenchus mucronatus* | Bmucfv | gatgcgtgtttagag gac | Bmucrv | ggaagaacgtagagca ca | Bmuc | aacaccgacccacccg ataa |
| *Mesocriconema xenoplax* | Mefv | cttgctcgtactggtc tg | Mxerv | cagcttctacaccgaga g | Mxe | cctgctcgtgctgttgtcaa |
| *Rotylenchus uniformis* | Runfv | acggaccaaggag tttag | Runrv | cccaggatcacacatca g | Run | caggggagaccttcactt tcatt |

The reaction mixture contains a buffer, a thermostable Taq polymerase, magnesium ions, primers and a probe. The concentration of the primers in the reaction mixture is 1-2 µM, of the probe 50100 nM, of magnesium ions 2-5 mM. The reaction is conducted in the primers annealing temperature 55-60°C, in reaction mixture volume 10-20 µl using from 35 to 40 reaction cycles. Identification of the products is done by comparing the amplification curves for the analyzed sample with the amplification curves for a blank sample.

The names of the primer and probe sequences correspond to consecutive sequence numbers: Bmucfv (SEQ ID NO: 1), Bmucrv (SEQ ID NO: 2), Bmuc (SEQ ID NO: 3), Mxefv (SEQ ID NO: 4), Mxerv (SEQ ID NO: 5), Mxe (SEQ ID NO: 6), Runfv (SEQ ID NO: 7), Runrv (SEQ ID NO: 8), Run (SEQ ID NO: 9).
- Fig. 1: shows amplification curves for *Bursaphelenchus mucronatus.*
- Fig. 2: shows amplification curves for *Mesocriconema xenoplax.*
- Fig. 3: shows amplification curves for *Rotylenchus uniformis.*

Examples of identification of each nematode species comprised in the kit are given below. In each case, the blank sample (deionized nuclease-free H₂O) and negative sample were subjected to identical isolation and amplification conditions. The negative sample material was DNA of a nematode species belonging to the same genus as the analyzed one, a mixture of equal amounts of DNA of nematodes belonging to the same species as the analyzed species or in case of no species belonging to the same genus, a species of the same family.

### Example 1

### Identification of nematodes of the Bursaphelenchus mucronatus species.

DNA was isolated using commercially available NucleoSpin Tissue XS kits for DNA isolation, from Macherey-Nagel. The sequences of the primers and the probe used in the reaction were the following:

| Primer/probe | Sequence |
|---|---|
| Bmucfv (SEQ ID NO: 1) | gatgcgtgtttagaggac |
| Bmucrv (SEQ ID NO: 2) | ggaagaacgtagagcaca |
| Bmuc (SEQ ID NO: 3) | aacaccgacccacccgataa |

The Real Time PCR reaction was conducted in a mixture with a volume of 20 µl in the RotorGene 6000 device from Qiagen using reagents from the LuminoCt qPCR ReadyMix kit (Sigma-Aldrich) in the following amounts:
- nuclease-free H₂O to 20 µl,
- 2 µl of 10.0 µM of each of the Bmucfv and Bmucrv primers,
- 1 µl of 4.0 µM of the Bmuc probe, labelled with JOE dye at the 5'-end and with HBQ1 quencher at the 3'-end,
- 10 µl of 2X LuminoCt qPCR ReadyMix (Sigma-Aldrich),
- 2 µl of DNA.

The reaction mixture was placed in a 20 µl test tube, placed in the RotorGene 6000 thermocycler rotor and amplification reaction was conducted in 40 cycles in the following conditions:

| Stage | | Temperature [C°] | Time [s] | Fluorescence |
|---|---|---|---|---|
| Pre-incubation | | 95 | 180 | - |
| Amplification | denaturation | 95 | 30 | - |
| | annealing | 55 | 30 | single |
| | synthesis | 72 | 30 | - |
| Cooling | | 40 | 20 | - |

The negative control was DNA of the *Bursaphelenchus crenati, Bursaphelenchus fraudulentus* and *Bursaphelenchus tiliae* nematodes.

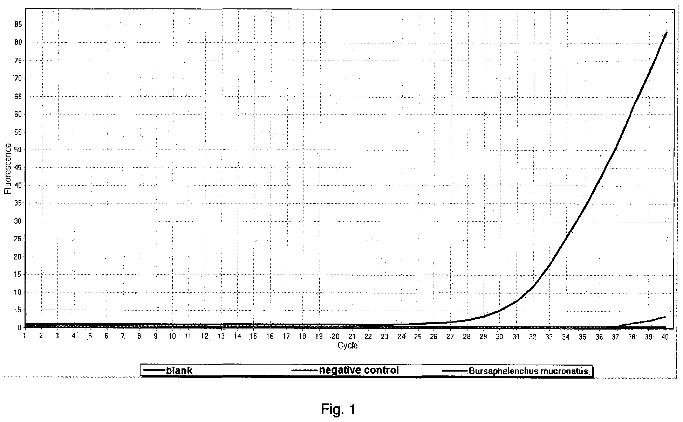

The amplification curves obtained as a result of the performed analysis are shown on Fig. 1.

### Example 2

### Identification of nematodes of the Mesocriconema xenoplax species.

The isolation and amplification conditions corresponded to those described in example 1. In the Real-Time PCR reaction the following primers and probe were used:

| Primer/probe | Sequence |
|---|---|
| Mxefv (SEQ ID NO: 4) | cttgctcgtactggtctg |
| Mxerv (SEQ ID NO: 5) | cagcttctacaccgagag |
| Mxe (SEQ ID NO: 6) | cctgctcgtgctgttgtcaa |

The negative control was DNA of the *Mesocriconema curvatum* and *Mesocriconema rusticum* nematodes.

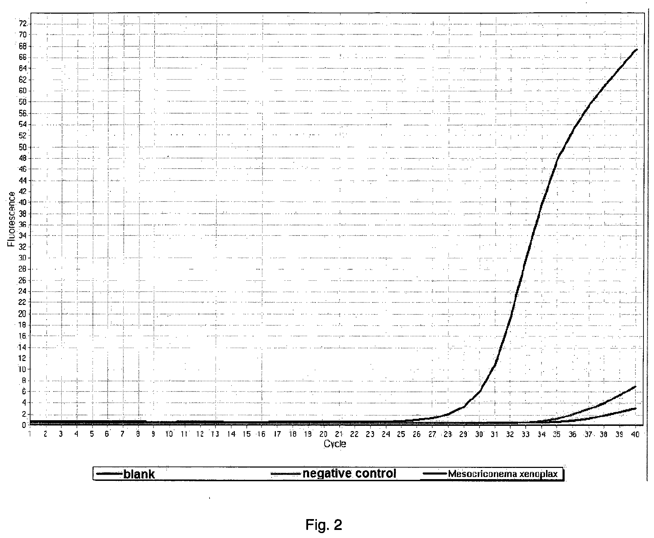

The amplification curves obtained as a result of the performed analysis are shown on Fig. 2.

### Example 3

### Identification of nematodes of the Rotylenchus uniformis species.

The isolation and amplification conditions corresponded to those described in example 1. In the Real-Time PCR reaction the following primers and probe were used:

| Primer/probe | Sequence |
|---|---|
| Runfv (SEQ ID NO: 7) | acggaccaaggagtttag |
| Runrv (SEQ ID NO: 8) | cccaggatcacacatcag |
| Run (SEQ ID NO: 9) | caggggagaccttcactttcatt |

The negative control was DNA of the *Rotylenchus agnetis, Rotylenchus buxophilus* and *Rotylenchus robustus* nematodes.

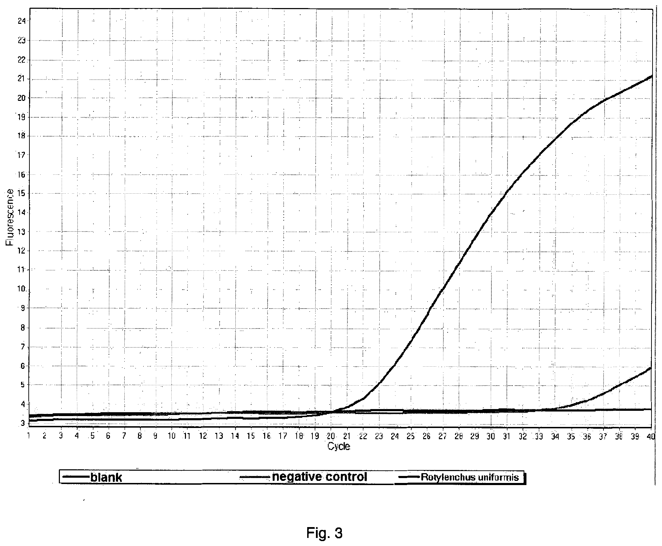

The amplification curves obtained as a result of the performed analysis are shown on Fig. 3.

The solution according to the invention allows detection of nematodes in soil within a few hours, including the stages of sample preparation, DNA isolation and Real-Time PCR reaction. The method according to the disclosure may be used for identification of the abovementioned nematode species regardless of the analyzed sample type, their origin and purpose, as well as developmental stage.

The proposed probe setup enables identification of the abovementioned nematode species in a Real Time PCR reaction, which is significantly more sensitive and rapid than other PCR methods. The use of Real Time PCR probes significantly increases reaction specificity in comparison to Real Time PCR with fluorescent dyes.
## Claims

1. A method of identification of nematodes in a soil sample, selected from the group consisting of the species *Bursaphelenchus mucronatus, Mesocriconema xenoplax* and *Rotylenchus uniformis,* the method comprising the following stages:
a) providing a soil sample containing nematodes for identification;
b) washing the nematodes off the soil;
c) DNA isolation;
d) conducting a Real-Time PCR reaction, using a pair of 3' and 5' primers and a probe;
e) comparing the amplification curve of the analyzed sample with amplification curves of a blank sample and negative control,
wherein for identification of one of the abovementioned species in the Real-Time PCR reaction, the used 3' and 5' primer and the probe suitable for the particular species are selected from the following list:
| Species | 5' primer | | 3' primer | | Probe | |
|---|---|---|---|---|---|---|
| | Name | Sequence | Name | Sequence | Name | Sequence |
| *Bursaphelenchus mucronatus* | Bmucfv | gatgcgtgttta gaggac | Bmucrv | ggaagaacgtagag caca | Bmuc | aacaccgacccaccc gataa |
| *Mesocriconema xenoplax* | Mxefv | cttgctcgtactg gtctg | Mxerv | cagcttctacaccgag ag | Mxe | cctgctcgtgctgttgtc aa |
| *Rotylenchus uniformis* | Runfv | acggaccaag gagtttag | Runrv | cccaggatcacacat cag | Run | caggggagaccttca ctttcatt |

2. A kit for identification of nematodes selected from the group consisting of the species *Bursaphelenchus mucronatus, Mesocriconema xenoplax* and *Rotylenchus uniformis,* according to the method of claim 1, comprising the 3' and 5' primers and the probe suitable for the particular species, selected from the following list:
| Species | 5' primer | | 3' primer | | Probe | |
|---|---|---|---|---|---|---|
| | Name | Sequence | Name | Sequence | Name | Sequence |
| *Bursaphelenchus mucronatus* | Bmucfv | gatgcgtgttta gaggac | Bmucrv | ggaagaacgtagagca ca | Bmuc | aacaccgacccaccc gataa |
| *Mesocriconema xenoplax* | Mxefv | cttgctcgtactg gtctg | Mxerv | Cagcttctacaccgag ag | Mxe | cctgctcgtgctgttgtc aa |
| *Rotylenchus uniformis* | Runfv | acggaccaag gagtttag | Runrv | Cccaggatcacacat cag | Run | caggggagaccttca ctttcatt |

## Patentansprüche

1. Verfahren zur Identifizierung von Nematoden in einer Bodenprobe, ausgewählt aus einer Gruppe bestehend aus den Arten *Bursaphelenchus mucronatus, Mesocriconema xenoplax* und *Rotylenchus uniformis,* wobei das Verfahren die folgenden Abschnitte umfasst:
a) Bereitstellen einer Bodenprobe mit Nematoden;
b) Herauswaschen der Nematoden aus der Bodenprobe;
c) Isolierung der DNA;
d) Durchführen einer Echtzeit-PCR unter Verwendung eines Paares von 3' und 5' Primern und einer Sonde;
e) Vergleichen der Amplifikationskurve der analysierten Probe mit den Amplifikationskurven einer Blindprobe und der Negativkontrolle,
wobei zur Identifizierung einer der oben genannten Spezies in der Echtzeit-PCR die verwendeten 3' und 5' Primer und die für die jeweilige Spezies geeignete Sonde aus der folgenden Liste ausgewählt wurden:
| Spezies | 5' primer | | 3' primer | | Sonde | |
|---|---|---|---|---|---|---|
| | Name | Sequence | Name | Sequence | Name | Sequence |
| *Bursaphelenchus mucronatus* | Bmucfv | gatgcgtgttta gaggac | Bmucrv | ggaagaacgtagag caca | Bmuc | aacaccgacccaccc gataa |
| *Mesocriconema xenoplax* | Mxefv | cttgctcgtactg gtctg | Mxerv | cagcttctacacc gag ag | Mxe | cctgctcgtgctgttgtc aa |
| *Rotylenchus uniformis* | Runfv | acggaccaag gagtttag | Runrv | cccaggatcacacat cag | Run | caggggagaccttca ctttcatt |

2. Set zur Identifizierung von Nematoden, ausgewählt aus einer Gruppe, bestehend aus den Spezies
*Bursaphelenchus mucronatus, Mesocriconema xenoplax* und *Rotylenchus uniformis,* gemäß
Verfahren nach Anspruch 1, umfassend die 3' und 5' Primer und die für die jeweilige Spezies geeignete Sonde, ausgewählt aus der folgenden Liste:
| Spezies | 5' primer | | 3' primer | | Sonde | |
|---|---|---|---|---|---|---|
| | Name | Sequence | Name | Sequence | Name | Sequence |
| *Bursaphelenchus mucronatus* | Bmucfv | gatgcgtgttta gaggac | Bmucrv | ggaagaacgtagagc a ca | Bmuc | aacaccgacccaccc gataa |
| *Mesocriconema xenoplax* | Mxefv | cttgctcgtact g gtctg | Mxerv | Cagcttctacaccga g ag | Mxe | cctgctcgtgctgttgtc aa |
| *Rotylenchus uniformis* | Runfv | acggaccaag gagtttag | Runrv | Cccaggatcacacat cag | Run | caggggagaccttca ctttcatt |

## Revendications

1. Un procédé d'identification des nématodes dans un échantillon de sol, choisi dans le groupe constitué des espèces *Bursaphelenchus mucronatus, Mesocriconema xenoplax* et *Rotylenchus uniformis,* ce procédé comprenant les étapes suivantes :
a) la fourniture d'un échantillon de sol contenant des nématodes pour identification ;
b) le lavage des nématodes du sol ;
c) l'isolement de l'ADN ;
d) la réalisation d'une réaction PCR en temps réel, en utilisant une paire d'amorces 3' et 5' et d'une sonde ;
e) la comparaison de la courbe d'amplification de l'échantillon analysé avec les courbes d'amplification d'un échantillon à blanc et d'un témoin négatif,
dans lequel, pour l'identification de l'une des espèces mentionnées ci-dessus dans la réaction PCR en temps réel, l'amorce 3' et 5' utilisée et la sonde appropriée pour l'espèce particulière sont choisies dans la liste suivante :
| Espèces | amorce 5' | | amorce 3' | | Sonde | |
|---|---|---|---|---|---|---|
| | Nom | Séquence | Nom | Séquence | Nom | Séquence |
| *Bursaphelenchus mucronatus* | Bmucfv | gatgcgtgttta gaggac | Bmucrv | ggaagaacgtagag caca | Bmuc | aacaccgacccaccc gataa |
| *Mesocriconema xenoplax* | Mxefv | cttgctcgtactg gtctg | Mxerv | cagcttctacaccgag ag | Mxe | cctgctcgtgctgttgtc aa |
| *Rotylenchus uniformis* | Runfv | acggaccaag gagtttag | Runrv | cccaggatcacacat cag | Run | caggggagaccttca ctttcatt |

2. Un kit pour l'identification des nématodes choisis dans le groupe constitué par les espèces *Bursaphelenchus mucronatus, Mesocriconema xenoplax* et *Rotylenchus uniformis,* selon le procédé de la revendication 1, comprenant les amorces 3' et 5' et la sonde appropriée pour les espèces particulières, choisies dans la liste suivante :
| Espèces | amorce 5' | | amorce 3' | | Sonde | |
|---|---|---|---|---|---|---|
| | Nom | Séquence | Nom | Séquence | Nom | Séquence |
| *Bursaphelenchus mucronatus* | Bmucfv | gatgcgtgttta gaggac | Bmucrv | ggaagaacgtagagc a ca | Bmuc | aacaccgacccaccc gataa |
| *Mesocriconema xenoplax* | Mxefv | cttgctcgtactg gtctg | Mxerv | Cagcttctacaccga g | Mxe | cctgctcgtgctgttgtc aa |
| *Rotylenchus uniformis* | Runfv | acggaccaag gagtttag | Runrv | Cccaggatcacacat cag | Run | cagggagaccttca ctttcatt |


<110> Muzeum i Instytut Zoologii Polskiej Akademii Nauk
<120> Method and kit for identification of nematodes, forest plant
   pests, by Real-Time PCR
<130> PZ/2798/PCT/AR
<150> P.410982
   <151> 2015-01-16
<160> 9
<170> PatentIn version 3.5
<210> 1
   <211> 18
   <212> DNA
   <213> Bursaphelenchus mucronatus
<400> 1
   gatgcgtgtt tagaggac 18
<210> 2
   <211> 18
   <212> DNA
   <213> Bursaphelenchus mucronatus
<400> 2
   ggaagaacgtagagcaca 18
<210> 3
   <211> 20
   <212> DNA
   <213> Bursaphelenchus mucronatus
<400> 3
   aacaccgacc cacccgataa 20
<210> 4
   <211> 18
   <212> DNA
   <213> Mesocriconema xenoplax
<400> 4
   cttgctcgta ctggtctg 18
<210> 5
   <211> 18
   <212> DNA
   <213> Mesocriconema xenoplax
<400> 5
   cagcttctac accgagag 18
<210> 6
   <211> 20
   <212> DNA
   <213> Mesocriconema xenoplax
<400> 6
   cctgctcgtg ctgttgtcaa 20
<210> 7
   <211> 18
   <212> DNA
   <213> Rotylenchus uniformis
<400> 7
   acggaccaag gagtttag 18
<210> 8
   <211> 18
   <212> DNA
   <213> Rotylenchus uniformis
<400> 8
   cccaggatca cacatcag 18
<210> 9
   <211> 23
   <212> DNA
   <213> Rotylenchus uniformis
<400> 9
   caggggagac cttcactttc att 23